(19) Europäisches Patentamt / European Patent Office / Office européen des brevets

(11) **EP 2 067 473 A2**

(12) **EUROPEAN PATENT APPLICATION**

(43) Date of publication:
**10.06.2009 Bulletin 2009/24**

(51) Int Cl.:
*A61K 31/205* (2006.01)   *A61P 9/10* (2006.01)

(21) Application number: **08170583.2**

(22) Date of filing: **03.12.2008**

| | |
|---|---|
| (84) Designated Contracting States:<br>**AT BE BG CH CY CZ DE DK EE ES FI FR GB GR HR HU IE IS IT LI LT LU LV MC MT NL NO PL PT RO SE SI SK TR**<br>Designated Extension States:<br>**AL BA MK RS**<br><br>(30) Priority: **05.12.2007 EP 07122360** | (71) Applicant: **Grindeks, a joint stock company Riga 1057 (LV)**<br><br>(72) Inventors:<br>• **Stonans, Ilmars**<br>  **LV-1057, Riga (LV)**<br>• **Kalvins, Ivars**<br>  **LV-1006, Riga (LV)** |

(54) **Medical use of 3-(2,2,2-trimethylhydrazinium) propionate salts**

(57)     The present invention relates to highly effective treatment of ischemic heart disease with 3-(2,2,2-trimethylhydrazinium) propionate dihydrogen phosphate.

**EP 2 067 473 A2**

## Description

Technical Field

The present invention relates to use of 3-(2,2,2-trimethylhydrazinium) propionate dihydrogen phosphate in the treatment of ischemic heart disease.

Background Art

Myocardiac infarction is a condition of irreversible necrosis of heart muscle that results from prolonged ischemia.

Myocardial infarction (heart attack) is a serious result of coronary artery disease. Myocardial infarction (MI) is the irreversible necrosis of heart muscle secondary to prolonged ischemia. A heart attack or myocardial infarction is a medical emergency in which the supply of blood to the heart is suddenly and severely reduced or cut off, causing the muscle to die from lack of oxygen. More than 1.1 million people experience a heart attack (myocardial infarction) each year, and for many of them, the heart attack is their first symptom of coronary artery disease. A heart attack may be severe enough to cause death or it may be silent. As many as one out of every five people have only mild symptoms or none at all, and the heart attack may only be discovered by routine electrocardiography done some time later.

A heart attack (myocardial infarction) is usually caused by a blood clot that blocks an artery of the heart. The artery has often already been narrowed by fatty deposits on its walls. These deposits can tear or break open, reducing the flow of blood and releasing substances that make the platelets of the blood sticky and more likely to form clots. Sometimes a clot forms inside the heart itself, then breaks away and gets stuck in an artery that feeds the heart. A spasm in one of these arteries causes the blood flow to stop.

3- (2,2,2-Trimethylhydrazinium) propionate dihydrate is known as compound with cardioprotective properties (this substance being known under its International Non-proprietary Name of Meldonium dihydrate). 3- (2,2,2-Trimethylhydrazinium) propionate is disclosed in US 4481218 (INST ORGANICHESKOGO SINTEZA) 06.11.1984

It is well known that 3- (2, 2,2-trimethylhydrazinium) propionate as dihydrate is widely used for controlling carnitine and gamma-butyrobetaine concentration ratio and consequently the speed of fatty acid beta-oxidation in the body DAMBROVA M., LIEPINSH E., KALVINSH I. I. Mildronate: cardioprotective action through carnitine-lowering effect. Trends in Cardiovascular Medicine,. 2002, vol.12, no.6, p.275-279.

Due to these properties, Meldonium dihydrate is extensively applied in medicine as an anti-ischemic, stress-protective and cardioprotective drug in treating various cardio- vascular diseases and other pathologies involving tissue ischemia KARPOV R.S., KOSHELSKAYA O.A., VRUBLEVSKY A.V., SOKOLOV A.A., TEPLYAKOV A.T., SKARDA I., DZERVE V., KLINTSARE D., VIOLS A., KALNINS U., KALVINSH I., MATVEYA L., URBANE D. Clinical Efficacy and Safety of Mildronate in Patients With Ischemic Heart Disease and Chronic Heart Failure. Kardiologiya. 2000, no.6, p. 69-74.. In the treatment of cardiovascular diseases the mechanism of action of 3-(2,2,2-trimethylhydrazinium)propionate based on limitation of carnitine biosynthesis rate and related long-chain fatty acid transport limitation through mitochondria membranes SIMKHOVICH B.Z., SHUTENKO Z.V., MEIRENA D.V., KHAGI K.B., MEZHAPUKE R.J., MOLODCHINA T.N., KALVINS I.J., LUKEVICS E. 3-(2,2,2-Trimethylhydrazinium) propionate (THP) - a novel gamma-butyrobetaine hydroxylase inhibitor with cardioprotective properties. Biochemical Pharmacology. 1988, vol.37, p.195-202., KIRIMOTO T., ASAKA N., NAKANO M., TAJIMA K., MIYAKE H., MATSUURA N.. Beneficial effects of MET-88, a $\gamma$-butyrobetaine hydroxylase inhibitor in rats with heart failure following myocardial infarction. European Journal of Pharmacology. 2000, vol.395, no.3, p.217-224.

Dihydrogen phosphate salt of Meldonium are disclosed in EP 1667960 A (JOINT STOCK COMPANY GRINDEKS) 14.06.2006 as more stable substance comparatively with Meldonium dihydrate.

Disclosure of Invention

As it was known what Meldonium dihydrate is used for treatment of Ischemic Heart Disease; however there are no data what Meldonium salts is used for treatment of Ischemic Heart Disease.

To our surprise, by using Meldonium dihydrogen phosphate in myocardial infarction and/or ischemia treatment it shows unexpected effect, and was more effective as Meldonium dihydrate in vivo myocardial infarction model. The dihydrogen phosphate salt of Meldonium, what is pharmaceutical acceptable salt, bioequivalent wit Meldonium dihydrate and should show the same medical effect like a Meldonium dihydrate, unexpectedly showed statistically better therapeutically effect than Meldonium dihydrate.

Best Mode for Carrying Out the Invention

The following examples further illustrate the invention.

Anti- ischemic activity

Experiments were conducted on adult male Wistar rats with initial weight of 300-350g. During the experiment, the animals were kept in Standard crates in groups of 8. The feed was a standardized diet R70 (LABFOR, Lactamin AB, Sweden). The room temperature was kept at 21-23°C, relative humidity at 65±10%, 12 hour light/darkness cycle.

The experimental procedures were carried out in accordance with the guidelines of the European Community and local laws and policies and were approved by Latvian Animal Protection Ethical Committee, the Food and Veterinary Service.

Myocardial infarction in vivo

Rats weighing approximately 300 g were randomly divided into 3 groups of 8 animals each. The first group received saline by mouth (control group); the second received 100 mg/kg Meldonium dihydrate by mouth, the third 100mg/kg Meldonium dihydrogen phosphate received for 14 days.

Rats were anesthetized with sodium pentobarbital (60 mg/kg i.p.). They were intubated and artificially respirated with rodent respirator (Rodent Ventilator 7025, Ugo Basile, Itally) with 15 mL/kg room air at a respiration rate of 55 breaths/min.

Thoracic was open on the left side of breastbone by cutting fourth and fifths, if necessary, ribs. The pericard was open and 5/0 polypropylene suture (Surgipro II, Syneture) was passed under the left anterior descending coronary artery and threaded through a small plastic tube to permit reversible occlusion of the coronary artery. Coronary flow was measured using an ultrasound flow detector (HSE) and PowerLab 8/30 system from ADInstruments.

Occlusion was performed by constricting threads through a plastic tube.

At the end of the 120 minute reperfusion, After reperfusion hearts were excised and retrogradely perfused via aorta at a constant pressure of 50 mm Hg, with oxygenated Krebs-Henseleit buffer ( content in mmol/L: NaCl 118, Ca $Cl_2$ 2.52, $MgCl_2$ 1.64, $NaHCO_3$ 24.88, $KH_2PO_4$ 1.18, glucose 10.0, EDTA 0.05) pH 7.3-7.5 at 37°C. Then after 10 min the left anterior descending coronary artery was relegated and the risk zone was delineated with 4 mL of 0.1% methylene blue solution in Krebs-Henseleit buffer infused via the aorta root. Hearts were sectioned transversely from the apex to the base of 2 mm thickness and incubated in 1 % triphenyl-tetrazolium chloride in phosphate buffer (pH 7.4, 37°C) for 10 minutes to stain viable tissue red and necrotic tissue white. Afterward, the right ventricle was cut off and photos of the left-ventricle slices were made with a Minolta 7D photo camera. Computerized planemetric analysis of photographs was performed using Image-Pro Plus 4.5.1 software to determine the area at risk (AR) and area of necrosis (AN) expressed as percentage of the left ventricle (LV). Obtained values were then used to calculate the infarct size (IS) as percentage of risk area according to formula:

$$IS(\%) = \frac{AN}{AR} \times 100$$

Results of myocardial infarction in vivo by administration with Meldonium dihydrate and Meldonium dihydrogen phosphate for 14 days are summarize in Table 1.

Table 1

| Results of myocardial infarction in vivo | | | |
|---|---|---|---|
| | Area of Risk/ left ventricle area | Area of Necrosis, % of Area at Risk | Infarct size, % of control |
| Control | 52.0±2.5 | 64.3±2.7 | 100.0±4.1 |
| Meldonium dihydrate | 58.0±2.2 | 64.0±2.7 | 99.0±4.2 |
| Meldonium dihydrogen phosphate | 57.0±3.0 | 50.9±2.6*,# | 79.0±4.1*,# |
| *p<0.01compared with the control group; #p<0.01 compared with Meldonium dihydrate group. | | | |

**Claims**

1. Use of 3-(2,2,2-trimethylhydrazinium)propionate dihydrogen phosphate for the manufacture of a medicament for the treatment of ischemic heart disease.

2. 3-(2,2,2-Trimethylhydrazinium)propionate dihydrogen phosphate for the treatment of ischemic heart disease.

3. Use of according to claim 1 or claim 2 where ischemic heart disease is myocardial infarction.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4481218 A

- EP 1667960 A

**Non-patent literature cited in the description**

- **DAMBROVA M. ; LIEPINSH E. ; KALVINSH I. I.** Mildronate: cardioprotective action through carnitine-lowering effect. *Trends in Cardiovascular Medicine,* 2002, vol. 12 (6), 275-279
- **KARPOV R.S. ; KOSHELSKAYA O.A. ; VRUBLEVSKY A.V. ; SOKOLOV A.A. ; TEPLYAKOV A.T. ; SKARDA I. ; DZERVE V. ; KLINTSARE D. ; VIOLS A. ; KALNINS U.** Clinical Efficacy and Safety of Mildronate in Patients With Ischemic Heart Disease and Chronic Heart Failure. *Kardiologiya.,* 2000, 69-74

- **SIMKHOVICH B.Z. ; SHUTENKO Z.V. ; MEIRENA D.V. ; KHAGI K.B. ; MEZHAPUKE R.J. ; MOLODCHINA T.N. ; KALVINS I.J. ; LUKEVICS E.** 3-(2,2,2,-Trimethylhydrazinium) propionate (THP) - a novel gamma-butyrobetaine hydroxylase inhibitor with cardioprotective properties. *Biochemical Pharmacology.,* 1988, vol. 37, 195-202
- **KIRIMOTO T. ; ASAKA N. ; NAKANO M. ; TAJIMA K. ; MIYAKE H. ; MATSUURA N.** Beneficial effects of MET-88, a γ-butyrobetaine hydroxylase inhibitor in rats with heart failure following myocardial infarction. *European Journal of Pharmacology,* 2000, vol. 395 (3), 217-224